(19) Europäisches Patentamt
European Patent Office
Office européen des brevets

(11) **EP 2 856 932 B1**

(12) **EUROPEAN PATENT SPECIFICATION**

(45) Date of publication and mention
of the grant of the patent:
**05.04.2017  Bulletin 2017/14**

(51) Int Cl.:
**A61B 3/16** (2006.01)

(21) Application number: **14187136.8**

(22) Date of filing: **30.09.2014**

(54) **Digital applanation tonometer**

Digitales Applanationstonometer

Tonomètre d'aplanation numérique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2013  GB 201317601**

(43) Date of publication of application:
**08.04.2015  Bulletin 2015/15**

(73) Proprietor: **Haag-Streit UK Limited
Harlow, Essex CM20 2TT (GB)**

(72) Inventors:
• **Johnson, Dean
Nr Canterbury, Kent CT4 7DB (GB)**

• **Henderson, Martin
Canterbury, Kent CT1 3PP (GB)**

(74) Representative: **Oxley, Rachel Louise et al
Mewburn Ellis LLP
City Tower
40 Basinghall Street
London EC2V 5DE (GB)**

(56) References cited:
**CN-U- 202 161 301        GB-A- 1 360 603
US-A1- 2004 210 123     US-A1- 2009 131 779**

EP 2 856 932 B1

**Description**

Field of the Invention

The present invention relates to applanation tonometers, particularly to applanation tonometers with a digital output.

Background of the Invention

In the fields of ophthalmology and optometry it is common practice to carry out measurements to determine the intraocular pressure of an eye of a patient. Such measurements are particularly important in relation to the diagnosis and routine monitoring of glaucoma diseases.

Determination of the intraocular pressure of a patient's eye can be carried out by various methods and techniques. These methods include contact methods where a device directly touches the eye of a patient to applanate (flatten) a portion of the eye, and non-contact methods whereby a portion of the eye is applanated without the device contacting the eye directly.

An example of a contact method is the Goldmann applanation tonometer, an example of which is disclosed in US 3,070,997 (F. Papritz, 1963). The Goldmann applanation tonometer has an applanation head which is slowly brought into contact with the anaesthetised eye (cornea). After contact has been made, the applanation area can be viewed and is made visible using fluorescein. The instrument uses a spring loaded feeler arm connected to the applanation head to apply a variable pressure to the cornea of the eye. The Goldmann applanation tonometer uses the Imbert-Fick law, where the tonometer measures the pressure required to applanate the cornea over a circular area of 3.06 mm. At this point, the surface tension force that results from the cornea is equal, but opposite to the force required to counteract corneal rigidity. The opposing forces of corneal rigidity and the tear film are roughly equal in a normal cornea and cancel each other out allowing the pressure within the eye to be inferred from the force applied.

US 3,952,585 and GB 1360603A (Perkins et al.), disclose an applanation tonometer including an applanation measurement head mounted onto an arm, which is coupled to a manual adjustment thumb wheel dial knob by means of a spiral spring. The thumb wheel dial knob is calibrated to derive the spring tension, when the applanation measurement head applies the required pressure to the eye.

US 5,012,812 (Stockwell, 1991) discloses an applanation tonometer which aims to improve on the accuracy of tonometers such as that disclosed in US 3,952,585 by incorporating a different mechanical configuration in which two spiral springs are located in series between a thumb wheel dial knob and an applanation arm via an intermediate spindle.

Both of the tonometers of US 3,952,585 and US 5,012,812 are fairly convenient to use in that they are self-contained and capable of being handheld. However, both rely on a mechanical readout such as a dial usually marked in steps of 2mmHg. This makes it difficult to quantify precise values for the recorded intraocular pressure.

In addition, the mechanical dial is difficult to read in the low-lighting conditions in which the device is generally used. It also introduces considerable user error and is time consuming, particularly where large numbers of repeat readings are taken.

Furthermore, calibration of the tonometers of US 3,952,585 and US 5,012,812 is also mechanical in nature so is time consuming.

The present invention aims to address these problems by providing an applanation tonometer which is more convenient to use and also offers a higher degree of accuracy. In addition, to provide a cost effective solution, the applanation tonometer of the present invention aims to solve these problems in such a way that it can be constructed by retrofitting a known self-contained mechanical tonometer such as that of US 3,952,585 and US 5,012,812 to include all of the features of the present invention.

US 2004/0210123 A1 (Davidson, 2004) and US 2009/0131779 A1 (Siegenthaler, 2009) each disclose Goldmann type tonometers with a digital output signal. However, the devices disclosed in US 3,070,997, US 2004/0210123 A1 and US 2009/0131779 A1 are required to be mounted on and used in conjunction with a Slit Lamp Microscope system. They are not capable of functioning as convenient self-contained, fully independent devices.

CN202161301 U also discloses an applanation tonometer for use in conjunction with a slit lamp microscope. The tonometer includes a potentiometer mounted on a gearwheel shaft.

Summary of the Invention

The invention is defined in independent claims 1 and 14 and the dependent claims 2 - 13.

According to a first aspect, the present invention aims to solve the above problems by providing an applanation tonometer comprising: a light source; a measurement head for applying an applanation pressure to a cornea; a control means for controlling the pressure applied by the measurement head to the cornea, the control means including a cam;

a sensor for detecting the rotational position of the cam, the sensor configured to produce an electrical output; and a microcontroller configured to convert the electrical output of the sensor into a digital output which corresponds to the pressure applied by the measurement head to the cornea; wherein the sensor is a rotary potentiometer attached to the cam.

In this way, an improved self-contained handheld applanation tonometer is provided which outputs a digital signal to give a more accurate reading of intraocular pressure.

Optional features of the invention will now be set out. These are applicable singly or in any combination with any aspect of the invention.

Preferably, at least a portion of the sensor directly contacts the cam.

The cam is preferably operated by a manual adjustment thumb wheel, the cam and the adjustment wheel preferably having a 1:1 rotation.

The microcontroller preferably includes an integrated analogue to digital converter configured to digitally encode the output of the sensor.

The maximum dimension of the sensor is preferably smaller than the maximum diameter of the cam. In this way, the amount of space taken up by the sensor is minimised. Thus, the sensor may easily be retrofitted onto a cam of a previously mechanical (i.e. non-digital) applanation tonometer. This considerably reduces manufacturing costs because the (digital) applanation tonometer can be made by retrofitting of a non-digital applanation tonometer.

Where a cam has an irregular shape, with more than one diameter, the maximum diameter of the cam is the largest possible length of the cam passing through the centre of the cam in a direction transverse to the rotational axis of the cam.

Preferably, the maximum lateral dimension of the sensor is not more than 12mm; the maximum lateral dimension of the sensor is the largest out of the length/width/diameter of the sensor in any direction transverse to the rotational axis of the cam.

Optionally, the sensor is 12mm square.

Preferably, the applanation tonometer further comprises a display means; wherein the display means includes a display screen and wherein the microcontroller is further configured to control the display of the digital output on the display screen.

Preferably, the display means includes a flexible PCB. In this way, the PCB is able to follow curves in an outer casing of the tonometer thereby optimising the use of space inside of the outer casing of the tonometer and providing more space for electronic components and circuitry. Such a circuit board provides various advantages over standard PCBs which are usually thick, fragile and restrictive. In particular, the flexible PCB facilitates the manufacture of the tonometer of the present invention by retrofitting of previously mechanical (non-digital) tonometers. Preferably, the flexible PCB is a double-sided flexible PCB. In this way, further space is provided for extra circuitry.

Preferably, the applanation tonometer includes a first PCB and a second PCB; wherein the first PCB is the flexible PCB which forms part of the display means and wherein the microcontroller is mounted onto the second PCB.

The second PCB may be flexible. Preferably the second PCB is less flexible than the first PCB. In this way, the second PCB is at least semi rigid providing a structure for the electrical components associated with data handling.

The second PCB preferably has a thickness of no more than 1mm. This thickness may preferably be no more than 0.5mm.

Optionally, the sensor (rotary potentiometer) is mounted onto the second PCB on the opposite side of the second PCB to the cam and the second PCB includes a hole through which a portion of the rotary potentiometer protrudes to contact the cam. In this way, space inside the tonometer is optimised as the first PCB can be located closer to the cam and therefore closer to the centre of the body of the tonometer where more space is available.

The second PCB is preferably laminated to prevent shorting.

Preferably, the microcontroller is configured to carry out a data handling operation independently from a data presentation operation; wherein: the data handling operation converts the electrical output of the sensor into a digital output corresponding to the pressure applied by the measurement head to the cornea; and the data presentation operation controls the display of the digital output on the display screen. In this way, the display parameters can be modified independently of the data-handling routine.

Optionally, the display screen is an integrated digital high contrast luminous display.

Alternatively, the display screen may be a liquid crystal display or a dot matrix.

Optionally, the screen is configured to display white characters on a black background. Such a configuration is particularly advantageous as it maximizes contrast whilst minimising ambient light emitted by the luminous display.

Optionally, the applanation tonometer may comprise a mechanical display in addition to the digital display screen.

Preferably, the control means includes a spring arrangement. The spring arrangement is preferably a pair of spiral springs arranged in series.

Preferably, the light is a Light Emitting Diode (an LED). Even more preferably, the light is a pair of LEDs.

Preferably the LED is a blue LED.

Preferably, the microcontroller further comprises a communications module.

Optionally, the communication module comprises a transmitter.

The communication module may comprise a USB data communications connection.

According to a second aspect of the present invention, there is provided applanation tonometer comprising: a light source; a measurement head for applying an applanation pressure to a cornea; a control means for controlling the pressure applied by the measurement head to the cornea, the control means including a cam; a sensor for detecting the rotation of the cam, the sensor configured to produce an electrical output; and a microcontroller configured to convert the electrical output of the sensor into a digital output which corresponds to the pressure applied by the measurement head to the cornea; wherein the sensor is a digital compass configured to detect the rotational position of the cam.

All of the optional features described above in relation to the first aspect should also be considered to be optional features of the second aspect.

According to a third aspect of the present invention, there is provided a method of manufacturing a digital applanation tonometer including the steps of: providing a mechanical applanation tonometer, the mechanical applanation tonometer comprising a measurement head for applying an applanation pressure to a cornea and a control means for controlling the pressure applied by the measurement head to the cornea; attaching a rotary potentiometer or a digital compass to the control means; inserting a display screen; and inserting a microprocessor configured to convert an electrical output of the potentiometer or digital compass to a digital output which corresponds to the pressure applied to the cornea and configured to control the display of the digital output on the display screen.

Preferably, the control means includes a cam and the rotary potentiometer/digital compass is located on the cam.

Further optional features of the measurement head, control means, potentiometer, digital compass, display screen and microprocessor are as described for the first and second aspects.

Further optional features of the invention are set out below.

Brief Description of the Drawings

Embodiments of the invention will now be described by way of example with reference to the accompanying drawings in which:

Figure 1 shows a cross sectional schematic diagram of an applanation tonometer according to the present invention;

Figure 2 shows a perspective drawing of an embodiment of the applanation tonometer of the present invention including a digital display screen;

Figure 3 shows a perspective drawing of an alternative embodiment of the applanation tonometer of the present invention including a digital display screen and a mechanical display;

Figure 4 shows a display means of an applanation tonometer according to the present invention;

Figure 5 shows a display means and outer casing of an applanation tonometer according to the present invention;

Figure 6 shows a schematic diagram of the applanation tonometer according to the present invention; and

Figure 7 shows a flow diagram of the software algorithm which controls the applanation tonometer of the present invention.

Detailed Description and Further Optional Features of the Invention

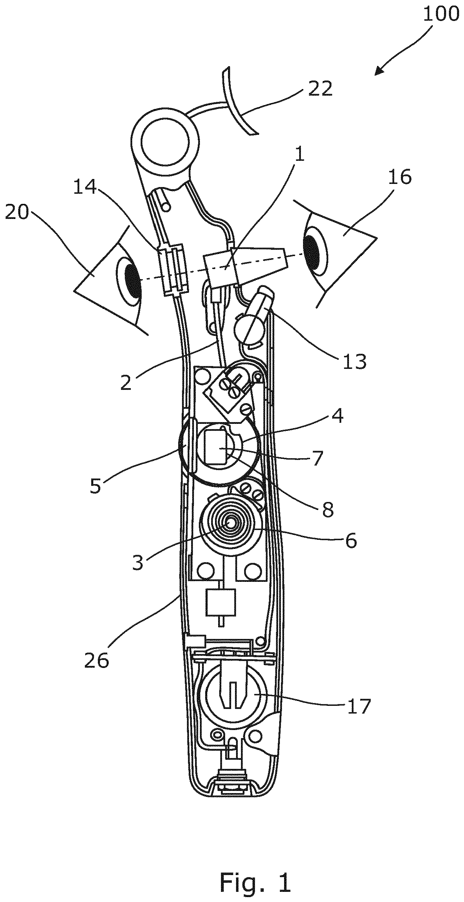

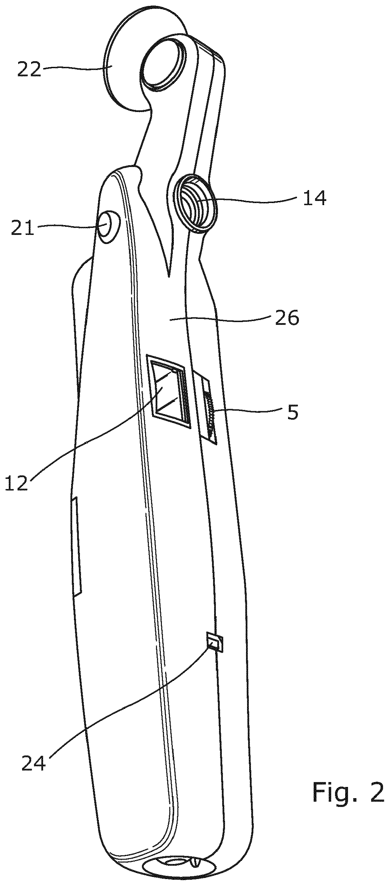

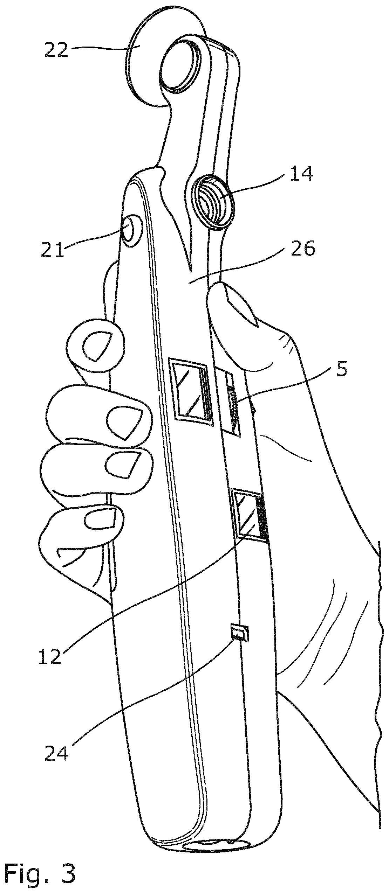

An applanation tonometer, 100 according to the present invention is described with reference to Figures 1, 2, 3 and 6. The applanation tonometer includes a light source 13, a measurement head 1 arranged to be capable of applying an applanation pressure to the cornea of a patient, a control means for controlling the pressure applied, a sensor 7 in the form of a rotary potentiometer and a microcontroller (not shown).

The control means includes a cam 8 onto which the sensor, 7 is connected.

The applanation tonometer, 100 is a self-contained (fully independent) applanation tonometer, the body of which is enclosed by an outer casing, 26. The outer casing has an ergonomic shape with curved edges.

The tonometer includes a balanced arm, 2 upon which the measurement head is mounted and a spindle, 3 pivoted in the body. The balance arm, 2 is rotatable and is mounted on the spindle, 3.

A manual adjustment thumb wheel 5, which can be rotated with respect to the outer casing, 26 operates the rotation of the cam. The balanced arm, 2 and applanation measuring head, 1 can be temporarily locked into place by means of a mechanical grip arrangement, 21, to enable routine replacement of the applanation measuring head, 1.

The control means also includes a spring arrangement comprising coil springs 4, 6 applies a torque on the balanced arm, 2 when the thumb wheel 5 is turned. The coil springs are located in series between the thumb wheel and the balanced arm.

This spring arrangement is described in more detail in US 5,012,812.

The sensor, 7 converts the mechanical reading from the adjustment thumb wheel, 5 to a digital reading. This method is non-invasive to the spring arrangement, 4, 6 of the control means which applies a torque on the balanced arm 2. This torque is directly proportionate to the rotary position (i.e. the angle from the zero-position) of the thumb-wheel, 5.

The sensor, 7 is a rotary potentiometer sensor, 7 arranged so that the output is first filtered, 9, then digitally encoded by means of an analogue to digital converter, 10 integrated into a microcontroller, 19, 11, which in turn reads the digital input, calculates the corresponding measurement and displays the recorded intraocular pressure via a display means 12. The microcontroller, 11 directly controls two blue low powered Light Emitting Diodes, 13 which are configured to illuminate the measurement head when the tonometer is in use, 1. This enables manual alignment and reading of intraocular pressure to be undertaken via a viewing eyepiece, 14.

The microcontroller, 11 includes a communication means to transmit the intraocular pressure reading to a PC via a communications module in the form of a Bluetooth module and/or USB data communications connection, 15. This enables intraocular pressure readings to be sent to a software based electronic patient record system, configured to monitor progression of the intraocular pressure over multiple readings. Furthermore, the communication module enables system diagnostics, testing and calibration to be undertaken.

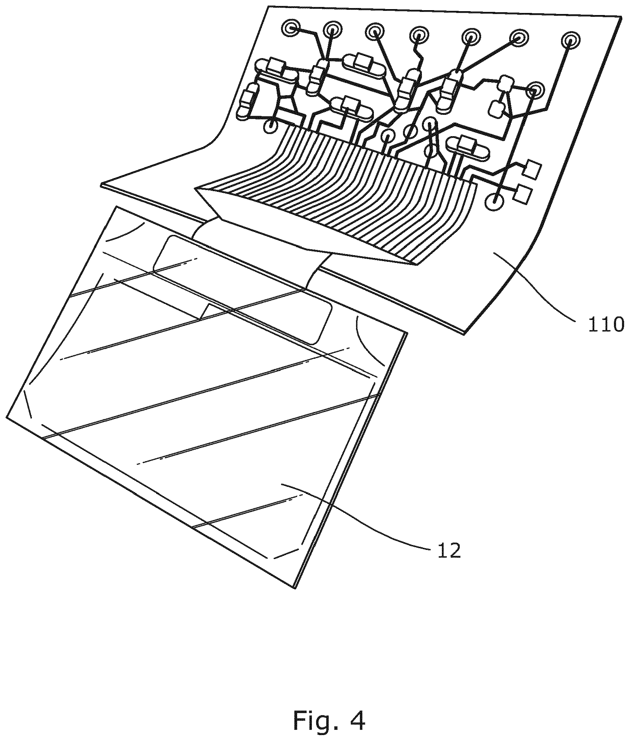

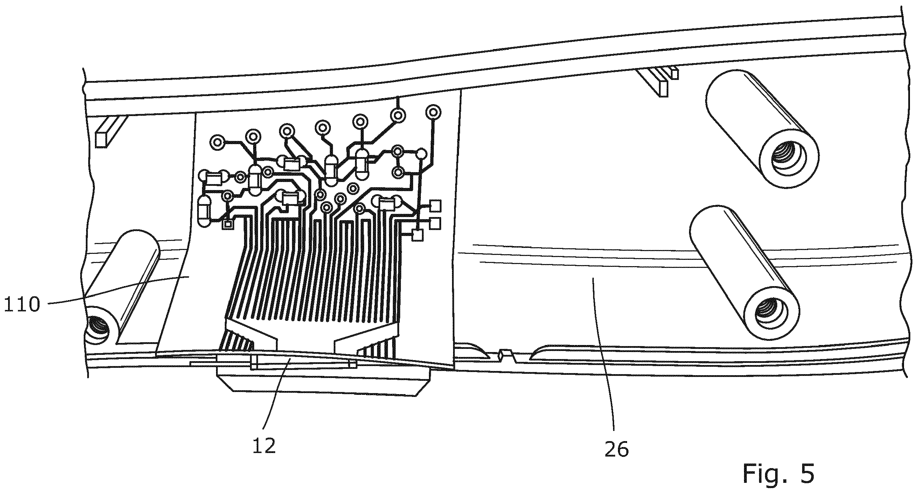

As shown in Figures 4 and 5, the display means includes a display screen, 12 and a flexible PCB 110. The outer casing 26 defines a window through which the display screen can be viewed.

The flexible double sided PCB is capable of being folded into a right angle as shown in Figure 4 which enables it to conform to the outer casing, 26 of the tonometer, forming a 'hugging' fit along the inner wall of the outer casing whereby the flexible PCB lies flush against the inner wall in order to provide an improved utilisation of space as shown in Figure 5.

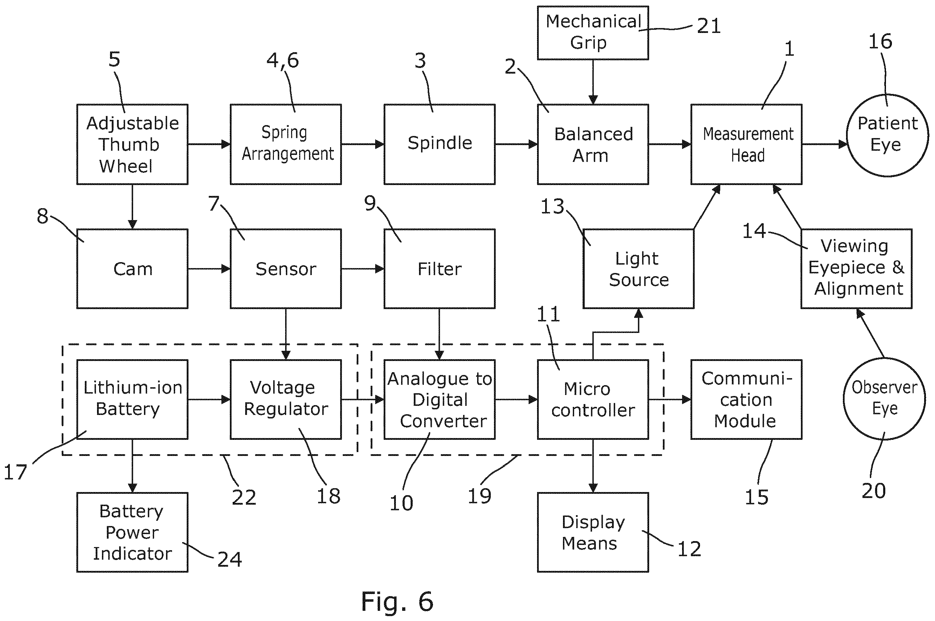

When not in use, the adjustable thumb-wheel is turned to a zero-position. When in use, the adjustable thumb-wheel, 5 is moved off the zero-position so that cam, 8 closes a switch, resulting in power being applied to the tonometer to operate and control the electronic elements. As shown in Figure 6, power is supplied via power source 22 located within the outer casing, 26, the power source, 22 comprising a rechargeable lithium-ion battery, 17 and voltage regulator, 18. The power source, 22 is controlled by the microcontroller, 11. In addition to the other electronic components required to generate and display the digital output, the power source also supplies power to the light source, 13. The adjustable thumb wheel therefore also controls the light source such that the lights are automatically 'on' when the tonometer is in use and the lights are automatically 'off' when the tonometer is in the zero position.

A battery/power indicator, 24 is configured to display the status of the battery, the indicator having three modes: battery charged, low battery charge and battery charging.

The sensor (in the form of a rotary potentiometer) produces a variable voltage output which is proportional to the rotational position of the cam as set out in Equation 1:

$$\frac{V_{pot}}{V_{DD}} = \frac{cam\ position_{current}}{cam\ position_{max}}$$

## Equation 1

Where: $V_{DD}$ is the Supply voltage; $V_{pot}$ is the output signal voltage.

The position can be measured using any units, as both sides of the equation are ratios and therefore dimensionless.

The position of the rotary potentiometer is first converted from a mechanical to an electrical form, and then from an electrical form to a digital form by means of analogue-to-digital conversion, 10. The method of analogue-to-digital conversion, 10 may be external to the microcontroller, or be included as part of the functionality within the microcontroller, 19. The process of analogue-to-digital conversion enables the mechanical position to be sampled and represented as a digital value.

The sensor, 7 can be calibrated by recording the output voltage zero-position with both a 2-gram and 5-gram calibration weight. The 2-gram weight optimises the system to approximately 20mmHg, whilst the 5-gram calibration weight enables the instrument's readings to be linearized across the entire range.

The reading is then calculated according to Equation 2:

$$reading = \frac{(-1)^d \times (V_{pot} - V_{zero})}{V_{weight}} \times M_{weight} \times 10mmHg, d \in [0,1]$$

## Equation 2

Where d is the direction of rotation, $V_{zero}$ is the output voltage from the rotary potentiometer when the instrument is in the zero-position, $V_{weight}$ is the output voltage recorded from the rotary potentiometer when the calibration weight is balanced on the prism, $V_{pot}$ is the output signal voltage and $M_{weight}$ is the mass in Newtons of the calibration weight.

An example of a suitable sensor is the Bourns™ 3382G-1-103G Potentiometer, a 12mm Rotary Position Sensor.

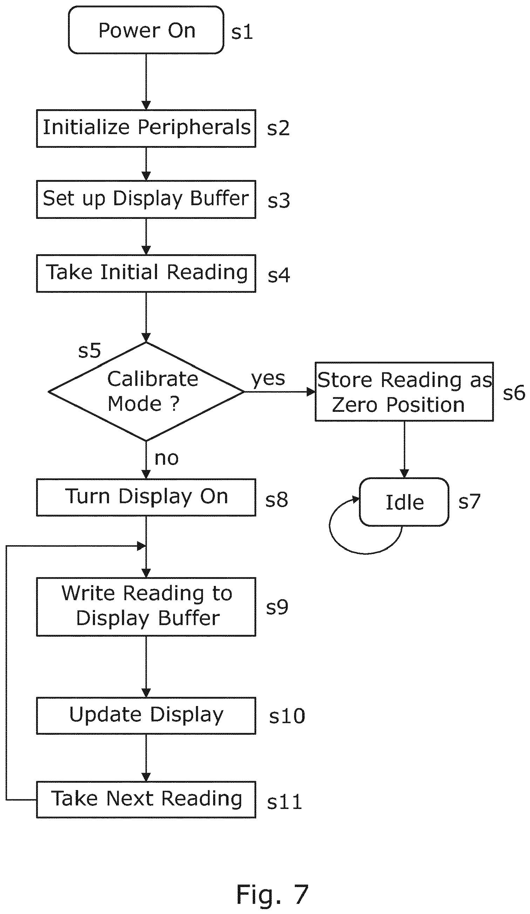

The microcontroller, 11 accepts information from the sensor, 7 regarding the position of the thumb wheel, 5, converting the data to an equivalent mmHg value and presenting the measured value on the display screen, 12. In order to achieve this functional and computational operation, the software produced to drive the microcontroller integrates the algorithm outlined in Figure 7.

The software structure written to control the microcontroller (11) functions is written in such a way as to separate the data-handling operation from the data-presentation operation. This approach enables the display (12) to be modified independent of microcontroller (11) data-handling routine.

Code in relation to the analogue-to-digital converter, 10 operation adopts a continuous approach, where the current mean value is derived from the past three sampled values. This approach digitally filters out noise which may be unavoidably introduced by the analogue-to-digital module, 10 itself.

Code in relation to the sensor 7, which assesses the analogue-to-digital converter, 10 interface, converts the raw 10-bit value of the digitised signal to mmHg by implementation of the formula described in Equation 2, where $V_{zero}$, $V_{pot}$ and $V_{weight}$ are replaced with their respective analogue-to-digital converted values.

Referring to Figure 7, the software structure includes an initialisation procedure s1-s4 triggered when the power is turned on, s1, including the steps of initialising peripherals, s2, setting up the display buffer, s3 and taking an initial reading, s4.

The software detects whether or not the tonometer is in the calibration mode, s5.

When the tonometer is set in the calibration mode during the calibration process, the reading is stored as the zero position, s6.

When the tonometer is in normal use (i.e. when not in calibration mode) the software algorithm steps followed include (in order): turning on the display, s8; writing the reading value to the display buffer, s9; updating the display, s10 and taking the next reading, s11.

According to embodiments of the second and third aspect of the present invention, the sensor, 7 is a digital compass where a diametric magnet is fixed to the thumb wheel, 5, where a break-out board housing the digital compass integrated circuit is positioned in adjacent proximity to the magnet. The digital compass sensor, 7 converts the mechanical reading from the thumb wheel, 5 to a digital reading, without the requirement for the sensor to be coupled to the rotary thumb wheel. This method is also non-invasive to the coil spring mechanism, 4, 6, which applies a torque on the balanced arm, 2, which is directly proportionate to the rotary position (angle from the zero-position) of the adjustable thumb-wheel, 5.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined in the claims.

## Claims

1. An applanation tonometer (100) comprising:

   a measurement head (1) for applying an applanation pressure to a cornea;
   a control means for controlling the pressure applied by the measurement head (1) to the cornea, the control means including a cam (8); and
   a sensor (7) for detecting the rotational position of the cam (8), the sensor (7) configured to produce an electrical output,

**characterised in that**:

the applanation tonometer (100) further comprises:

a light source (13); and
a microcontroller (11) configured to convert the electrical output of the sensor (7) into a digital output which corresponds to the pressure applied by the measurement head (1) to the cornea, and

the sensor (7) is a rotary potentiometer attached to the cam (8), the rotary potentiometer having a maximum lateral dimension that is smaller than the maximum diameter of the cam (8).

2. The applanation tonometer (100) of claim 1, wherein the maximum lateral dimension of the rotary potentiometer (7) is not more than 12mm.

3. The applanation tonometer (100) of claim 1 or 2, further comprising a display means; wherein the display means includes a display screen (12) and wherein the microcontroller (11) is further configured to control the display of the digital output on the display screen (12).

4. The applanation tonometer (100) of claim 3, wherein the display means includes a flexible PCB (110).

5. The applanation tonometer (100) of claim 4, wherein the flexible PCB (110) is a double-sided flexible PCB.

6. The applanation tonometer (100) of claim 4 or claim 5, further comprising a second PCB onto which the microcontroller (11) is mounted.

7. The applanation tonometer (100) of any one of claims 3 to 6, wherein the microcontroller (11) is configured to carry out a data handling operation independently from a data presentation operation; wherein
the data handling operation converts the electrical output of the sensor (7) into a digital output corresponding to the pressure applied by the measurement head (1) to the cornea; and
the data presentation operation controls the display of the digital output on the display screen (12).

8. The applanation tonometer (100) of any one of claims 3 to 7, wherein the display screen (12) is an integrated digital high contrast luminous display.

9. The applanation tonometer (100) of any one of the preceding claims, wherein the control means includes a spring arrangement.

10. The applanation tonometer (100) of any one of the preceding claims, wherein the light source (13) is an LED.

11. The applanation tonometer (100) of any one of the preceding claims, wherein the microcontroller (11) further comprises a communications module.

12. The applanation tonometer (100) of claim 11, wherein the communication module comprises a transmitter.

13. The applanation tonometer (100) of claim 11 or claim 12, wherein the communication module comprises a USB data communications connection (15).

14. A method of manufacturing a digital applanation tonometer (100) including the steps of:

providing a mechanical applanation tonometer, the mechanical applanation tonometer comprising a measurement head (1) for applying an applanation pressure to a cornea and a control means comprising a cam (8) for controlling the pressure applied by the measurement head (1) to the cornea;
attaching a rotary potentiometer (7) to the cam (8), the rotary potentiometer (7) having a maximum lateral dimension that is smaller than the maximum diameter of the cam (8);
inserting a display screen (12); and
inserting a microprocessor (11) configured to convert an electrical output of the potentiometer (7) to a digital output which corresponds to the pressure applied to the cornea and configured to control the display of the digital output on the display screen (12).

**Patentansprüche**

1. Applanationstonometer (100), umfassend:

   einen Messkopf (1), um einen Applanationsdruck auf eine Cornea auszuüben;
   ein Steuermittel, um den vom Messkopf (1) auf die Cornea ausgeübten Druck zu steuern, wobei das Steuermittel einen Nocken (8) umfasst; und
   einen Sensor (7) zum Detektieren der Drehposition des Nockens (8), wobei der Sensor (7) konfiguriert ist, um ein elektrisches Ausgangssignal zu produzieren; **dadurch gekennzeichnet, dass**
   das Applanationstonometer (100) ferner Folgendes umfasst:

   eine Lichtquelle (13) sowie
   eine Mikrosteuereinheit (11), welche konfiguriert ist, um das elektrische Ausgangssignal des Sensors (7) in ein digitales Ausgangssignal umzuwandeln, das dem Druck entspricht, der vom Messkopf (1) auf die Cornea ausgeübt wird;

   und dass der Sensor (7) ein Drehpotentiometer ist, welches an dem Nocken (8) befestigt ist, wobei das Drehpotentiometer eine maximale seitliche Dimension aufweist, die kleiner als der maximale Durchmesser des Nockens (8) ist.

2. Applanationstonometer (100) nach Anspruch 1, worin die maximale seitliche Dimension des Drehpotentiometers (7) nicht mehr als 12 mm ist.

3. Applanationstonometer (100) nach Anspruch 1 oder 2, welches ferner ein Anzeigemittel umfasst; worin das Anzeigemittel einen Anzeigebildschirm (12) umfasst und worin die Mikrosteuereinheit (11) ferner konfiguriert ist, um die Anzeige des digitalen Ausgangssignals auf dem Anzeigebildschirm (12) zu steuern.

4. Applanationstonometer (100) nach Anspruch 3, worin das Anzeigemittel eine flexible gedruckte Leiterplatte (110) umfasst.

5. Applanationstonometer (100) nach Anspruch 4, worin die flexible gedruckte Leiterplatte (110) eine doppelseitige, flexible gedruckte Leiterplatte ist.

6. Applanationstonometer (100) nach Anspruch 4 oder Anspruch 5, welches ferner eine zweite gedruckte Leiterplatte umfasst, auf welcher die Mikrosteuereinheit (11) montiert ist.

7. Applanationstonometer (100) nach einem der Ansprüche 3 bis 6, worin die Mikrosteuereinheit (11) konfiguriert ist, um unabhängig von einem Datenpräsentationsvorgang einen Datenhandhabungsvorgang durchzuführen; worin der Datenhandhabungsvorgang das elektrische Ausgangssignal des Sensors (7) in ein digitales Ausgangssignal umwandelt, das dem Druck entspricht, der vom Messkopf (1) auf die Cornea ausgeübt wird, und der Datenpräsentationsvorgang die Anzeige des digitalen Ausgangssignals auf dem Anzeigebildschirm (12) steuert.

8. Applanationstonometer (100) nach einem der Ansprüche 3 bis 7, worin der Anzeigebildschirm (12) ein integrierter digitaler Leuchtbildschirm mit hohem Kontrast ist.

9. Applanationstonometer (100) nach einem der vorangehenden Ansprüche, worin das Steuermittel eine Federanordnung umfasst.

10. Applanationstonometer (100) nach einem der vorangehenden Ansprüche, worin die Lichtquelle (13) eine LED ist.

11. Applanationstonometer (100) nach einem der vorangehenden Ansprüche, worin die Mikrosteuereinheit (11) ferner ein Kommunikationsmodul umfasst.

12. Applanationstonometer (100) nach Anspruch 11, worin das Kommunikationsmodul ein Sendeelement umfasst.

**13.** Applanationstonometer (100) nach Anspruch 11 oder Anspruch 12, worin das Kommunikationsmodul eine USB-Datenkommunikationsverbindung (15) umfasst.

**14.** Verfahren zur Herstellung eines digitalen Applanationstonometers (100), welches die folgenden Schritte umfasst:

Bereitstellen eines mechanischen Applanationstonometers, wobei das mechanische Applanationstonometer einen Messkopf (1) zum Ausüben eines Applanationsdrucks auf eine Cornea sowie ein Steuermittel umfasst, welches einen Nocken (8) umfasst, um den vom Messkopf (1) auf die Cornea ausgeübten Druck zu steuern;
Befestigen eines Drehpotentiometers (7) am Nocken (8), wobei das Drehpotentiometer (7) eine maximale seitliche Dimension aufweist, die kleiner als der maximale Durchmesser des Nockens (8) ist;
Einfügen eines Anzeigebildschirms (12); und
Einfügen eines Mikroprozessors (11), welcher konfiguriert ist, um ein elektrisches Ausgangssignal des Potentiometers (7) in ein digitales Ausgangssignal umzuwandeln, das dem auf die Cornea ausgeübten Druck entspricht, und welcher konfiguriert ist, um die Anzeige des digitalen Ausgangssignals auf dem Anzeigebildschirm (12) zu steuern.

**Revendications**

**1.** Tonomètre d'aplanation (100), comprenant :

une tête de mesure (1) pour appliquer une pression d'aplanation à une cornée ;
des moyens de commande pour commander la pression appliquée par la tête de mesure (1) à la cornée, les moyens de commande comprenant une came (8) ; et
un capteur (7) pour détecter la position de rotation de la came (8), le capteur (7) étant configuré pour produire une sortie électrique,
**caractérisé en ce que** :

le tononomètre d'aplanation (100) comprend en outre :

une source de lumière (13) ; et
une micro-commande (11) configurée pour convertir la sortie électrique du capteur (7) en une sortie numérique qui correspond à la pression appliquée par la tête de mesure (1) à la cornée, et
le capteur (7) est un potentiomètre rotatif fixé à la came (8), le potentiomètre rotatif ayant une dimension latérale maximale qui est inférieure au diamètre maximum de la came (8).

**2.** Tonomètre d'aplanation (100) selon la revendication 1, dans lequel la dimension latérale maximale du potentiomètre rotatif (7) n'est pas supérieure à 12 mm.

**3.** Tonomètre d'aplanation (100) selon la revendication 1 ou 2, comprenant en outre des moyens d'affichage ; dans lequel les moyens d'affichage comprennent un écran d'affichage (12) et dans lequel la micro-commande (11) est en outre configurée pour commander l'affichage de la sortie numérique sur l'écran d'affichage (12).

**4.** Tonomètre d'aplanation (100) selon la revendication 3, dans lequel les moyens d'affichage comprennent une carte de circuit imprimé (PCB) souple (110).

**5.** Tonomètre d'aplanation (100) selon la revendication 4, dans lequel la PCB souple (110) est une PCB souple double face.

**6.** Tonomètre d'ablation (100) selon la revendication 4 ou la revendication 5, comprenant en outre une seconde PCB sur laquelle la micro-commande (11) est montée.

**7.** Tonomètre d'ablation (100) selon l'une quelconque des revendications 3 à 6, dans lequel la micro-commande (11) est configurée pour exécuter une opération de traitement de données indépendamment d'une opération de présentation de données ; dans lequel
l'opération de traitement de données convertit la sortie électrique du capteur (7) en une sortie numérique correspondant à la pression appliquée par la tête de mesure (1) à la cornée ; et
l'opération de présentation de données commande l'affichage de la sortie numérique sur l'écran d'affichage (12).

**EP 2 856 932 B1**

8. Tonomètre d'aplanation (100) selon l'une quelconque des revendications 3 à 7, dans lequel l'écran d'affichage (12) est un affichage numérique intégré à fort contraste lumineux.

9. Tononomètre d'aplanation (100) selon l'une quelconque des revendications précédentes, dans lequel les moyens de commande comprennent un agencement à ressort.

10. Tonomètre d'aplanation (100) selon l'une quelconque des revendications précédentes, dans lequel la source de lumière (13) est une diode électroluminescente (LED).

11. Tonomètre d'ablation (100) selon l'une quelconque des revendications précédentes, dans lequel la micro-commande (11) comprend en outre un module de communication.

12. Tonomètre d'aplanation (100) selon la revendication 11, dans lequel le module de communication comprend un émetteur.

13. Tonomètre d'ablation (100) selon la revendication 11 ou la revendication 12, dans lequel le module de communication comprend une connexion de communications de données USB (15).

14. Procédé de fabrication d'un tonomètre d'aplanation numérique (100) comprenant les étapes consistant à :

fournir un tonomètre d'aplanation mécanique, le tononomètre d'aplanation mécanique comprenant une tête de mesure (1) pour appliquer une pression d'aplanation à une cornée et des moyens de commande comprenant une came (8) pour commander la pression appliquée par la tête de mesure (1) à la cornée ;
fixer un potentiomètre rotatif (7) à la came (8), le potentiomètre rotatif (7) ayant une dimension latérale maximale qui est inférieure au diamètre maximum de la came (8) ;
insérer un écran d'affichage (12) ; et
insérer un microprocesseur (11) configuré pour convertir une sortie électrique du potentiomètre (7) en une sortie numérique qui correspond à la pression appliquée à la cornée, et configuré pour commander l'affichage de la sortie numérique sur l'écran d'affichage (12).

**10**

Fig. 1

22 —

21 —

14

26

5

12

24

Fig. 2

Fig. 3

Fig. 4

Fig. 5

Fig. 6

EP 2 856 932 B1

Power On s1

Initialize Peripherals s2

Set up Display Buffer s3

Take Initial Reading s4

s5 Calibrate Mode ? — yes → Store Reading as Zero Position s6

no

Turn Display On s8

Idle s7

Write Reading to Display Buffer s9

Update Display s10

Take Next Reading s11

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3070997 A, F. Papritz
- US 3952585 A
- GB 1360603 A, Perkins
- US 5012812 A, Stockwell
- US 20040210123 A1, Davidson
- US 20090131779 A1, Siegenthaler
- CN 202161301 U